(19) Europäisches Patentamt
European Patent Office
Office européen des brevets

(11) EP 1 170 377 A2

(12) **EUROPÄISCHE PATENTANMELDUNG**

(43) Veröffentlichungstag:
**09.01.2002 Patentblatt 2002/02**

(51) Int Cl.[7]: **C12Q 1/26**

(21) Anmeldenummer: **01114732.9**

(22) Anmeldetag: **22.06.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **05.07.2000 DE 10032633**

(71) Anmelder: **Bayer Aktiengesellschaft**
**51368 Leverkusen (DE)**

(72) Erfinder:
- **Schallner, Otto, Dr.**
  **40789 Monheim (DE)**
- **Zitzmann, Werner, Dr.**
  **51381 Leverkusen (DE)**
- **Tietjen, Klaus-Günther, Dr.**
  **40764 Langenfeld (DE)**

(54) **Verfahren zum Auffinden von Hemmstoffen der Protoporphyrinogen-Oxidase**

(57) Die vorliegende Erfindung betrifft ein Verfahren zum Auffinden von Substanzen, die mit dem Enzym Protoporphyrinogen-Oxidase (PPO) wechselwirken, sowie ein Verfahren zum Testen der Wechselwirkung einer Substanz mit PPO. Des Weiteren betrifft die Erfindung Reagenzien für diese Verfahren, ein entsprechendes Test-System und PPO-Hemmstoffe, die durch die erfindungsgemäßen Verfahren identifiziert werden.

EP 1 170 377 A2

## Beschreibung

Die vorliegende Erfindung betrifft ein Verfahren zum Auffinden von Substanzen, die mit dem Enzym Protoporphyrinogen-Oxidase (PPO) wechselwirken, sowie ein Verfahren zum Testen der Wechselwirkung einer Substanz mit PPO. Des Weiteren betrifft die Erfindung Reagenzien für diese Verfahren, ein entsprechendes Test-System und PPO-Hemmstoffe, die durch die erfindungsgemäßen Verfahren identifiziert werden.

Unerwünschtes Pflanzenwachstum kann durch die Verwendung von Herbiziden verhindert werden. Die Ansprüche an Herbizide sind dabei hinsichtlich ihrer Wirksamkeit, Kosten und Umweltverträglichkeit stetig angestiegen. Es existiert deshalb ein Bedarf an neuen Substanzen, die zu leistungsfähigen neuen Herbiziden entwickelt werden können.

Herbizide haben in der Regel bestimmte Angriffspunkte im Stoffwechsel der Pflanze und wirken in vielen Fällen dadurch, dass bestimmte essentielle Stoffwechselwege der Pflanze gehemmt werden. In vielen Fällen hemmen oder blockieren herbizide Wirkstoffe Enzyme, die bestimmte Teilreaktionen solcher Stoffwechselwege als Biokatalysatoren erst ermöglichen. Die pflanzlichen Enzyme oder - allgemeiner formuliert - Proteine, welche die biochemische Grundlage für die Wirkung von Herbiziden bzw. von Substanzen mit potentiell herbizider Wirkung bilden, werden als Target-Proteine bezeichnet.

Einen besonders wichtigen Ansatzpunkt (Target) für Substanzen mit herbizider oder wachstumsregulierender Wirkung bilden die Enzyme des Biosynthesewegs für Porphyrine. Dies gilt besonders auch für das Enzym Protoporphyrinogen-Oxidase (PPO), das in zwei zellulären Formen vorkommt. Eine dieser beiden Formen befindet sich in den Chloroplasten der Pflanzen, die andere Form tritt in den Mitochondrien auf.

Das Enzym PPO katalysiert im Rahmen des Biosynthesewegs der Prophyrine die Oxidation von Protoporphyrinogen IX zu Protoporphyrin IX (Matringe et al. 1989, Biochem. J. 260, 231). Porphyrine sind bei einer Vielzahl von für die Pflanze wichtigen biochemischen Reaktionen von essentieller Bedeutung. Hierzu gehören u.a. die Biosynthese der Chlorophylle, die im Zusammenhang mit der Photosynthese der Pflanzen eine zentrale Funktion haben, sowie die Biosynthese des Häms, das an weiteren biologisch wichtigen Vorgängen, wie z.B. der Zellatmung, beteiligt ist. Häm ist ein Co-Faktor von Hämoglobin, Cytochromen, p450 Oxygenasen, Peroxidasen und Katalasen.

Der Wirkungsmechanismus von Herbiziden mit Wirkort PPO beruht vermutlich auf einer phototoxischen Reaktion. Bei Hemmung des Enzyms akkumuliert dessen Substrat Protoporphyrinogen IX in den Chloroplasten. Möglicherweise tritt diese Verbindung unter den genannten Bedingungen ins Cytosol aus, wo sie durch eine Peroxidase zu Protoporphyrin IX oxidiert wird. Protoporphyrin IX kann bei der Belichtung zur Bildung von Singulettsauerstoff und weiterer reaktiver Sauerstoffspezies führen. Infolge von Lipidperoxidation und daraus resultierenden Membranschäden kommt es dann zum schnellen Absterben der pflanzlichen Zellen (Lee et al. 1993, Plant Physiol. 102, 881).

Hemmstoffe des Enzyms PPO mit herbizider Wirkung können einer Reihe unterschiedlicher Substanzklassen zugeordnet werden, wie z.B. Diphenylether, Oxadiazole, cyclische Imide, Phenylpyrazole und Pyridinderivate (Duke et al. 1991, Weed Sci. 39, 465; Nandihalli et al. 1992, Pesticide Biochem. Physiol. 43, 193; Yanase und Andoh 1989, Pesticide Biochem. Physiol. 35, 70).

PPO-Hemmstoffe mit herbizider Wirkung kann man beispielsweise dadurch auffinden, dass man Substanzen direkt an Pflanzen austestet. Häufig werden diese Tests unter Gewächshausbedingungen durchgeführt. Durch entsprechend optimierte Verfahren ist es möglich, unter solchen Bedingungen eine Vielzahl von Substanzen auf ihre biologische Wirkung zu untersuchen. Ob auf diese Weise gefundene herbizid wirksame Verbindungen tatsächlich die PPO als Target haben, lässt sich letztlich nur durch enzymatische Studien mit isoliertem PPO-Enzym nachweisen.

Um den Durchsatz an zu testenden Substanzen zu erhöhen, wurden Tests mit miniaturisierten pflanzlichen Systemen bzw. Teilen von Pflanzen und pflanzlichen Zellkulturen entwickelt und in verstärktem Ausmaß durchgeführt. Selbst mit solchen miniaturisierten Testsystemen kann es aber mitunter schwierig sein, einen hinreichenden Probendurchsatz mit gut reproduzierbaren Testergebnissen zu erzielen.

Eine Alternative zu den oben genannten Methoden bietet das Screening mit biochemischen Testverfahren. Typische biochemische Verfahren umfassen in der Regel eine Isolierung und eine gegebenenfalls daran anschließende Reinigung des Enzyms aus geeignetem - im Fall von Herbizid-Targets - pflanzlichen Material. Eine weitere Möglichkeit besteht darin, das Enzym durch Klonierungsverfahren zugänglich zu machen. In diesem Fall wird das für das Target kodierende Gen identifiziert und in einem geeigneten heterologen System exprimiert. Geeignete Expressionssysteme sind z.B. Bakterien, Hefen, Säugerzellen, Insektenzellen sowie Pflanzen. Das Target-Protein kann daraufhin aufgereinigt und für spezifische Testverfahren eingesetzt werden.

Unabhängig davon, ob das Enzym direkt aus Pflanzen bzw. pflanzlichen Zellkulturen oder vergleichbaren Systemen gewonnen wird, oder aber mittels gentechnischer Verfahren im weitesten Sinn hergestellt wird, wird in der Regel ein biochemischer Aktivitätstest des Enzyms durchgeführt. Bei einem konventionellen enzymatischen Test wird die Bildung eines Produkts im Verlauf der enzymatisch katalysierten Reaktion zeitabhängig mit Hilfe geeigneter Messmethoden quantifiziert. Alternativ hierzu wird in manchen Fällen die Abnahme eines Edukts im Verlauf der enzymatischen Reaktion bestimmt. Die Messgröße besteht bei derartigen Methoden in der Regel in einer messbaren Änderung der Absorption oder Fluoreszenz der Produkte bzw. der Edukte einer solchen Reaktion. In vielen Fällen wird die Reaktion auch mit radiochemischen Methoden verfolgt. Anstelle der direkten Messung der enzymatischen Aktivität kann ein biochemisches Screening nach neuen Wirkstoffen auch mit Hilfe von Bindungstests durchgeführt werden. In diesem Fall beruht die Detektion der Hemmstoffe nicht auf der Bestimmung der Hemmung einer enzymatischen Reaktion wie bei den oben genannten Ansätzen, sondern auf einer Analyse des Bindungsverhaltens von Testsubstanzen. Grundlage dieses Ansatzes ist die empirisch belegte Tatsache, dass Hemmstoffe enzymatischer Reaktionen in der Mehrzahl der Fälle an das Enzym binden müssen, um das entsprechende Enzym inhibieren zu können.

Zur Analyse der Bindung von Substanzen an Enzyme und andere Proteinmoleküle, wie z.B. Rezeptoren, existiert inzwischen ein Vielzahl von Verfahren (siehe z.B. Hulme 1990, Receptor Biochemistry, IRL Press). Grundsätzlich zu unterscheiden sind hierbei Verfahren, bei denen markierte Verbindungen eingesetzt werden von solchen, bei denen eine solche Markierung nicht notwendig ist. Ein Nachteil solcher Verfahren besteht jedoch darin, dass es sich häufig um nicht-homogene Methoden handelt. In vielen Fällen müssen der freie und der an das Target-Protein gebundene Ligand physikalisch getrennt werden, sofern nicht eine direkte Detektion im homogenen System möglich ist.

Eine Aufgabe der vorliegenden Erfindung war es daher, ein Verfahren zu entwickeln, mit welchem bei einem möglichst hohen Durchsatz an Testverbindungen Substanzen aufgefunden werden können, die mit PPO wechselwirken und somit potentielle herbizide Wirkstoffe repräsentieren.

Die Aufgabe wurde gelöst durch die Bereitstellung eines Verfahrens zum Auffinden von Substanzen, die mit dem Enzym PPO wechselwirken und welches die folgenden Schritte umfasst:

a) Herstellen von Mischungen, die (i) PPO, (ii) eine bei geeigneter Bestrahlung fluoreszierende Substanz, die mit PPO in Wechselwirkung treten kann, und (iii) eine zu testende Substanz oder ein Gemisch aus zu testenden Substanzen in unterschiedlichen Konzentrationen umfassen,

b) Bestrahlen der Mischungen mit linear polarisiertem Licht einer geeigneten Wellenlänge, das die fluoreszierende Substanz zur Emission von Licht anregt, und

c) Messen der Fluoreszenzpolarisationswerte oder der Anisotropiewerte des emittierten Lichts.

Das Verfahren beruht auf der Messung der Änderung der Fluoreszenzpolarisation in einem Testsystem, welches bei Verdrängung einer niedermolekularen fluoreszierenden Substanz (Tracer) von der PPO durch eine zu testende Substanz erfolgt. Durch die hohe Empfindlichkeit von Fluoreszenzpolarisationstests ist es möglich, bereits geringe Konzentrationen von Substanzen, die mit der PPO wechselwirken, nachzuweisen. Eine Verringerung des Fluoreszenzpolarisationswertes bei steigender Konzentration der zu testenden Substanz oder des Gemisches aus zu testenden Substanzen zeigt eine Wechselwirkung einer oder mehrerer zu testenden Substanzen mit der PPO an. Anstelle der Fluoreszenzpolarisation kann auch die Anisotropie des Messansatzes analysiert und zur Auswertung der Experimente herangezogen werden. Fluoreszenzpolarisation und Anisotropie sind in diesem Zusammenhang fünktionell äquivalente Methoden (vgl. J.R.Lakowicz, Principles of Fluorescence Spectroscopy, 2.Auflage, Kluver Academic/Plenum Publishers, 1999).

Das Phänomen der Fluoreszenzpolarisation ist bereits seit Perrin (1926) bekannt, der die Entdeckung machte, dass nach Anregung eines fluoreszierenden Moleküls mit polarisiertem Licht das emittierte Fluoreszenzlicht ebenfalls in einer bestimmten Ebene polarisiert ist, wenn dieses Molekül zwischen Excitation und Emission stationär ist (siehe F. Perrin 1926: "Polarisation de la lumiere de fluorescence. Vie moyenne des molecules dans l'état excité", Seiten 390-401). Insgesamt hat die Fluoreszenzpolarisation (bzw., wie oben erwähnt, die Anisotropie) bis vor wenigen Jahren im Bereich des Screenings nach neuen Wirkstoffen nur sehr begrenzte Anwendung gefunden (siehe hierzu den Artikel von J. R. Sportsman et al. 1997: "Fluorescence Polarization" in High Throughput Screening, Herausgeber J. P. Devlin, Verlag Marcel Dekker). Durchaus anders war und ist die Situation im medizinisch-diagnostischen Bereich (siehe hierzu I. Hemmilä 1991: "Applications of Fluorescence in Immunoassays", Wiley Interscience). Häufig handelt es sich bei den im Diagnostikbereich bzw. in der klinischen Chemie eingesetzten Geräten um Küvettengeräte, die in der Regel für den hohen Probendurchsatz, wie er im Bereich des Wirkstoff-Screenings notwendig ist, nur wenig geeignet sind. Mit der Verfügbarkeit leistungsfähiger und sensitiver Plattenlesegeräte hat sich die Situation geändert, so dass inzwischen Anwendungen in Biochemie und Molekularbiologie (siehe Checovich W., Bolger R. und Burke T. 1995, Nature 375, 254-256) in breitem Umfang möglich geworden sind. Solche zur Messung der Fluoreszenzpolarisation bzw. der Anisotropie geeigneten Plattenlesegeräte für Mikrotiterplatten finden inzwischen auch Verwendung beim Wirkstoff-Screening, z.B. im Bereich der Auffindung neuer pharmazeutischer Wirkstoffe (siehe den oben genannten Artikel von J. R. Sportsman).

Eine weitere Aufgabe der vorliegenden Erfindung war es, ein Verfahren zu entwickeln, mit welchem getestet werden kann, ob eine Substanz mit dem Enzym PPO wechselwirkt oder nicht.

Diese Aufgabe wurde gelöst durch die Bereitstellung eines Verfahrens zum Testen, ob eine Substanz mit

dem Enzym Protoporphyrinogen-Oxidase (PPO) wechselwirkt, und welches die folgenden Schritte umfasst:

a) Herstellen von Mischungen, die (i) PPO und (ii) eine bei geeigneter Bestrahlung fluoreszierende Substanz in unterschiedlichen Konzentrationen umfassen,

b) Bestrahlen der Mischungen mit linear polarisiertem Licht einer geeigneten Wellenlänge, das die fluoreszierende Substanz zur Emission von Licht anregt, und

c) Messen der Fluoreszenzpolarisationswerte oder der Anisotropiewerte des emittierten Lichts.

Wenn somit die Vermutung besteht, dass eine bestimmte Substanz mit PPO in Wechselwirkung treten kann, lässt sich eine solche Wechselwirkung mit dem erfindungsgemäßen Verfahren dadurch nachweisen, dass man die zu testende Substanz mit einem geeigneten Fluoreszenzfarbstoff markiert - falls die zu testende Substanz nicht bereits per se fluoreszierende Eigenschaften aufweist - und das erhaltene Konjugationsprodukt (im Beispielteil als Tracer bezeichnet) in unterschiedlichen Konzentrationen mit PPO in Kontakt bringt. Eine Erhöhung des Fluoreszenzpolarisationswertes bei fallender Konzentration der fluoreszierenden Substanz zeigt eine Wechselwirkung mit der PPO an, da entsprechend einer fallenden Konzentration der fluoreszierenden, zur Bindung an das Targetprotein befähigten Substanz (des Tracers) ein zunehmend größerer Teil dieser Verbindung an das PPO-Molekül gebunden vorliegt. Aufgrund des entsprechend kleineren Anteils an freier, nicht an die PPO gebundener fluoreszierender Substanz (Tracer) steigen die gemessenen Fluoreszenzpolarisations- bzw. Anisotropiewerte.

Bevorzugt wird in die erfindungsgemäßen Verfahren pflanzliche PPO eingesetzt.

Besonders bevorzugt stammt die pflanzliche PPO aus Gerste oder Hafer, ganz besonders bevorzugt aus Etioplasten der Gerste. Die Gewinnung des Enzymmaterials aus Etioplasten hat den Vorzug, dass unter diesen Bedingungen ein vergleichsweise geringerer Hintergrund an die Messung möglicherweise störenden Verbindungen, zum Beispiel an Chlorophyllen, im Extrakt vorliegt.

Man kann für die erfindungsgemäßen Verfahren Pflanzenzellextrakte, die PPO enthalten, verwenden. Die Herstellung solcher Pflanzenextrakte erfolgt gemäß üblichen Standardverfahren. Das Pflanzenmaterial wird in der Regel homogenisiert, wozu verschiedene Verfahren eingesetzt werden können, wie zum Beispiel mechanische Homogenisierung, Ultraschallmethoden und andere Aufschlussverfahren, die für pflanzliches Material geeignet sind (vgl. hierzu Deutscher 1990, Guide to Protein Purification, Methods in Enzymology 182).

Die PPO kann jedoch auch biochemisch aus Pflanzenzellextrakten isoliert bzw. gereinigt werden. Durch entsprechende Verfahren kann ein besonders geeignetes Enzymmaterial hergestellt werden. Ein Beispiel dafür ist der Einsatz der Dialyse, die es ermöglicht, den Anteil an Verbindungen zu reduzieren, welche die in den erfindungsgemäßen Verfahren durchgeführten Messungen stören oder ungünstig beeinflussen können. Hierzu gehört auch die Reduktion der Viskosität der Enzympräparation und damit auch des Messansatzes, etwa durch eine Verringerung des Saccharosegehalts der Enzympräparation.

Weitere geeignete Verfahren, wie verschiedene chromatographische Reinigungsverfahren sowie Fällungs- und Extraktionsverfahren, die zu einer Anreicherung der PPO oder zu einer Abreicherung von die Polarisationsmessung oder deren Selektivität störenden Komponenten des Enzymextrakts führen können, finden sich in gängigen Publikationen aus dem Bereich der Proteinbiochemie (vgl. beispielsweise Deutscher 1990, Guide to Protein Purification, Methods in Enzymology 182; Marshak et al. 1996, Strategies for Protein Purification and Characterization, Cold Spring Harbor Lab., New York).

Eine weitere Möglichkeit besteht darin, das für PPO codierende Gen in heterologen Wirtszellen zu exprimieren und die PPO in Form eines Zellextrakts oder in gereinigter Form einzusetzen. Geeignete DNA, die für pflanzliche PPO codiert, ist in der WO 95/34659 beschrieben. Die Auswahl von geeigneten Promotoren zur Expression der für PPO codierenden DNA liegt im fachmännischen Können und ist davon abhängig, ob zur Expression pro- oder eukaryotische Zellen oder zellfreie Systeme verwendet werden. Beispiele für heterologe Promotoren sind der frühe oder späte Promotor des SV40, des Adenovirus oder des Cytomegalovirus, das lac-System, das trp-System, die Haupt-Operator- und Promotorregionen des Phagen lambda, die Kontrollregionen des fd-Hüllproteins, der Promotor der 3-Phosphoglyceratkinase, der Promotor der Sauren Phosphatase und der Promotor des $\alpha$-Mating-Faktors der Hefe. Als Vektoren können alle in molekularbiologischen Laboratorien verwendete Plasmide, Phasmide, Cosmide, YACs oder künstliche Chromosomen verwendet werden. Als Wirtszellen eignen sich sowohl prokaryotische Zellen, wie Bakterien der Gattungen Bacillus, Pseudomonas, Streptomyces, Streptococcus, Staphylococcus, vorzugsweise E. coli, als auch eukaryotische Zellen, wie Hefen, Säuger-, Insekten- oder Pflanzenzellen. Bevorzugte eukaryotische Wirtszellen sind u.a. Insektenzelllinien wie Sf9, die im Rahmen des Baculovirus-Expressionssystems als Wirtszellen eingesetzt werden können.

Ein schnelles Verfahren zum Isolieren von durch Wirtszellen synthetisierter PPO beginnt mit der Expression eines Fusionsproteins, wobei der Fusionspartner auf einfache Weise affinitätsgereinigt werden kann. Der Fusionspartner kann beispielsweise Glutathion S-Transferase sein. Das Fusionsprotein kann dann an einer Glutathion-Affinitätssäule gereinigt werden. Der Fusionspartner kann bei Bedarf durch partielle proteolytische Spaltung beispielsweise an Linkem zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere Modifikationen können für die Anreicherung und Reinigung klonierter Proteine in besonderem Mass nützlich sein, beispielsweise die Klonierung und Expression von mit His-Tags versehener PPO. Hierbei wird die Isolierung über entsprechende Affinitätsmethoden besonders erleichtert (vgl. Firmenschriften z.B. der Firmen Novagen, Qiagen und Amersham Pharmacia; zu Klonierungs- und Expressionsmethoden allgemein : Sambrook et al. 1989, Molecular Cloning, A Laboratory Handbook, 2.Aufl., Cold Spring Harbor Lab., New York).

Geeignete Reinigungsverfahren basieren auf präparativer Elektrophorese, FPLC, HPLC (z.B. unter Anwendung von Gelfiltrations-, Umkehrphasen- oder leicht hydrophoben Säulen, Gelfiltration, differentieller Präzipitation, Ionenaustausch-Chromatographie und Affinitätschromatographie, inklusive der Nickel-Chelatchromatographie und verwandte Methoden. Diese Methoden können zur Isolierung und Reinigung von PPO aus pflanzlichem oder tierischen Zellmaterial sowie aus Bakterien, Hefen oder Pilzen eingesetzt werden.

Bei der in die erfindungsgemäßen Verfahren einzusetzenden Substanz, die bei geeigneter Bestrahlung fluoresziert und die mit PPO in Wechselwirkung treten kann handelt es sich bevorzugt um Liganden der PPO, natürliche PPO-Substrate, natürliche Produkte der PPO-Enzymreaktion oder um herbizide wirksame PPO-Hemmstoffe.

Die Substanzen können bereits selbst über geeignete Fluoreszenzeigenschaften verfügen oder mit einem Fluoreszenzfarbstoff markiert sein.

Die fluoreszierende Substanz bzw. der Fluoreszenzfarbstoff, mit dem eine Substanz, die mit PPO in Wechselwirkung treten kann, markiert ist, sollte vorzugsweise ein Anregungsmaximum im Bereich von 250 bis 750 nm, ein Emissionsmaximum im Bereich von 300 bis 900 nm und eine Rotationsrelaxationszeit im Bereich von 1 bis 10 Nanosekunden besitzen. Die fluoreszierende Substanz bzw. der Fluoreszenzfarbstoff sollte vorzugsweise Eigenschaften aufweisen, die zu Polarisationswerten zwischen 0 und 400 mP führen. Beispiele solcher für die Markierung geeigneter Farbstoffe sind die Farbstoffe Fluorescein, Fluorescein-Deriyate, wie Fluoresceinisothiocyanat, Rhodamin, Dansyl-Derivate, wie Dansylchlorid, Cumarine bzw. Cumarin-Derivate, wie Umbelliferon, Fluorophore aus der Gruppe der Alexa-Fluoreszenzfarbstoffe (ALEXA Dyes, Fa. Molecular Probes Inc., Eugene, USA), wie [1,10-Dihydro-2,2,10,10-tetramethyl-4,8-bis(sulfomethyl)-2H-pyrano[3,2-g: 5,6-g']diquinolin-6-yl]-benzoesäure, 6-[2-Carboxyphenyl]-1,2,10,11 -tetrahydro-1,2,2,10,10,11-hexamethyl-4,8-bis(sulfomethyl)-pyrano[3,2-g:5,6-g']diquinolin-13-iumsalz, 6-[2-Carboxy-5-[(carboxymethyl)thio]-3,4,6-trichlorphenyl]-1,2,3,4,8,9,10,11 1-octahydro-2,2,4,8,10,10-hexamethyl-12,14-disulfo-pyrano[3,2-g:5,6-g']diquinolin-13-iumsalz, 5-(4-Carboxyphenyl)-1,2,3,7,8,9-hexahydro-2,3,3,7,7,8-hexamethyl-10,12-disulfo-pyrano[3,2-f:5,6-f']diindol-11-iumsalz, 3,6-Diamino-9-(2-carboxy-phenyl)-4,5-disulfo-xanthyliumsalz, 8,8-Dimethyl-2-oxo-6-(sulfomethyl)-4-(trifluormethyl)-2H-pyrano[3,2-g]quinoline-9(8H)-hexansäure, 7-Amino-3-methylcarboxy-4-methyl-2-oxo-2H-1-benzopyran-6-sulfonsäure, 2-(2,7-Dichlor-9,9a-dihydro-6-hydroxy-3-oxo-3H-xanthen-9-yl)-benzoesäure, 2-(2,7-Difluor-9,9a-dihydro-6-hydroxy-3-oxo-3H-xanthen-9-yl)-benzoesäure, 2-(2,4,5,7-Tetrabrom-9,9a-dihydro-6-hydroxy-3-oxo-3H-xanthen-9-yl)-benzoesäure, 2-(2,7-Dichlor-4-nitro-9,9a-dihydro-6-hydroxy-3-oxo-3H-xanthen-9-yl)-benzoesäure, 2-(9,9a-Dihydro-6-hydroxy-3-oxo-2,4,5,7-tetrajod-3H-xanthen-9-yl)-benzoesäure, 2-(4,5,6-trihydroxy-3-oxo-3H-xanthen-9-yl)-benzoesäure, sowie die jeweiligen Derivate davon, Fluorophore aus der Gruppe der CyDyes-Fluoreszenzfarbstoffe (Fa. Amersham Life Sciences, UK) sowie aus der Gruppe der Bodipy-Fluoreszenzfarbstoffe (Fa. Molecular Probes Inc., Eugene, USA).

Bevorzugt wird als Fluoreszenzfarbstoff Fluorescein verwendet.

Besonders bevorzugt weist die fluoreszierende Substanz die folgende Struktur auf:

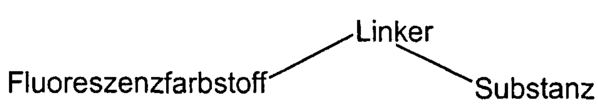


wobei

"Linker" für eine jeweils geradkettige oder verzweigte, jeweils gesättigte oder ungesättigte, jeweils gegebenenfalls substituierte, jeweils an einem Ende mit der Substanz und am anderen Ende mit dem Fluoreszenzfarbstoff verknüpfte Kohlenwasserstoffkette steht, welche jeweils am Anfang oder am Ende oder innerhalb der Kette eine oder mehrere der nachstehenden Hetero-Komponenten enthalten kann:

$$Q^1, CQ^2, CQ^2\text{-}Q^1, Q^1\text{-}CQ^2, Q^1\text{-}CQ^2\text{-}Q^1, SO, SO_2$$

wobei jeweils $Q^1$ und $Q^2$ für O, S oder NH stehen,
oder für eine jeweils gesättigte oder ungesättigte, jeweils gegebenenfalls substituierte an einem Ende mit der Substanz und am anderen Ende mit dem Fluoreszenzfarbstoff verknüpfte, carbocyclische oder heterocyclische Gruppierung steht, und
"Substanz"
für eine Substanz steht, welche mit PPO in Wechselwirkung treten kann.
"Fluoreszenzfarbstoff' steht bevorzugt für eine Farbstoff-Gruppierung, welche durch die nachstehende Formel gekennzeichnet ist,

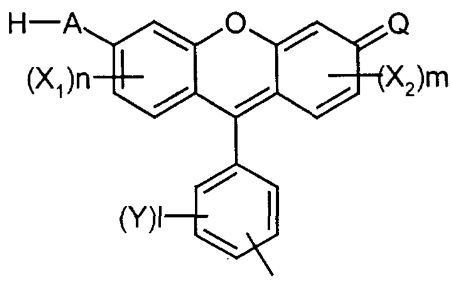

worin

A für O oder NH steht,

Q für O, S oder NH steht,

$X^1$ für Wasserstoff, Halogen, Nitro, Hydroxy, Carboxy (COOH), Sulfo ($SO_3H$) oder Alkyl steht,

$X^2$ für Wasserstoff, Halogen, Nitro, Hydroxy, Carboxy (COOH), Sulfo ($SO_3H$) oder Alkyl steht,

Y für Halogen, Alkyl, Carboxy (COOH) oder Sulfo ($SO_3H$) steht,

l für die Indizes 0 bis 4 steht, und

m, n für die Indizes 0 bis 3 steht.

A steht bevorzugt für O.

Q steht bevorzugt für O.

$X^1$ steht bevorzugt für Wasserstoff, Fluor, Chlor, Brom oder Alkyl mit 1 bis 4 Kohlenstoffatomen.

$X^2$ steht bevorzugt für Wasserstoff, Fluor, Chlor, Brom oder Alkyl mit 1 bis 4 Kohlenstoffatomen.

$X^1$ steht besonders bevorzugt für Wasserstoff, Fluor, Chlor, Methyl oder Ethyl.

$X^2$ steht besonders bevorzugt für Wasserstoff, Fluor, Chlor, Methyl oder Ethyl.

Die Kohlenwasserstoffkette des Linkers weist bevorzugt bis zu 10 Kohlenstoffatome auf.
Die carbocyclische oder heterocyclische Gruppierung des Linkers weist bevorzugt bis zu 10 Kohlenstoffatome und gegebenenfalls bis zu 5 Stickstoffatome und gegebenenfalls 1 oder 2 Sauerstoff- oder Schwefelatome auf.
Die Substanz weist bevorzugt die folgende Struktur auf:

$$Z^1\text{-}A^1\text{-}Z^2\text{-}$$

wobei

$A^1$ für eine Einfachbindung, O oder S steht,

$Z^1$ für eine carbocyclische oder heterocyclische Gruppierung mit jeweils bis zu 10 Kohlenstoffatomen und gegebenenfalls bis zu 5 Stickstoffatomen und gegebenenfalls 1 oder 2 Sauerstoff- oder Schwefelatomen steht, und

$Z^2$ für eine carbocyclische oder heterocyclische Gruppierung mit jeweils bis zu 10 Kohlenstoffatomen und gegebenenfalls bis zu 5 Stickstoffatomen und gegebenenfalls 1 oder 2 Sauerstoff- oder Schwefelatomen steht.

$A^1$ steht bevorzugt für eine Einfachbindung.

$Z^1$ steht bevorzugt für eine der nachstehenden Gruppierungen

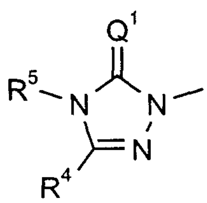
$(Z^1)$

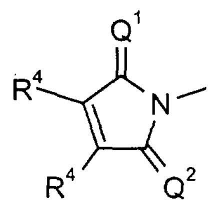
$(Z^2)$

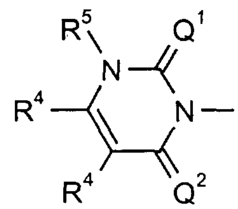
$(Z^3)$

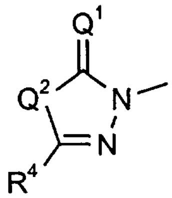
$(Z^4)$

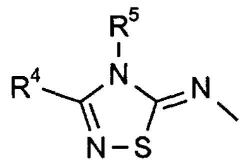
$(Z^5)$

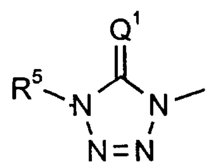
$(Z^6)$

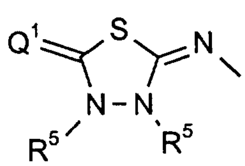
$(Z^7)$

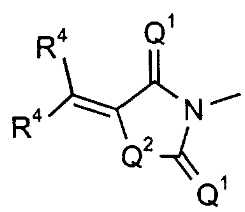
$(Z^8)$

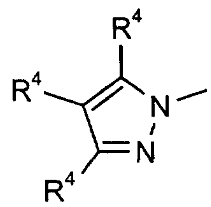
$(Z^9)$

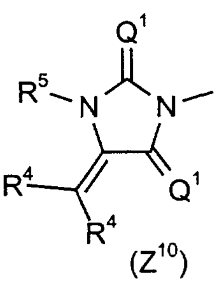
$(Z^{10})$

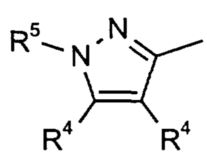
$(Z^{11})$

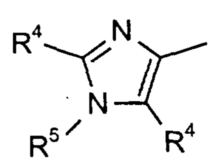
$(Z^{12})$

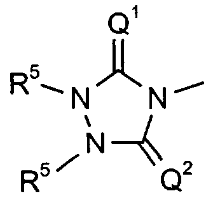
($Z^{13}$)

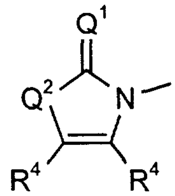
($Z^{14}$)

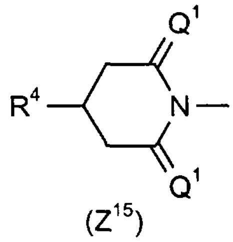
($Z^{15}$)

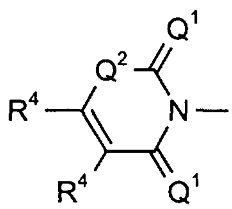
($Z^{16}$)

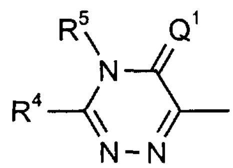
($Z^{17}$)

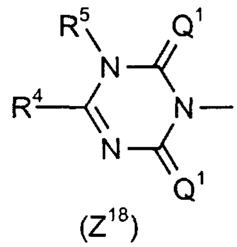
($Z^{18}$)

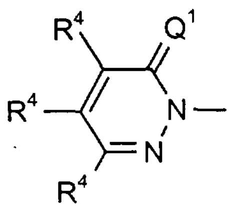
($Z^{19}$)

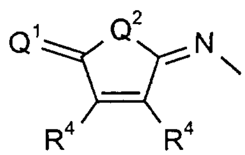
($Z^{20}$)

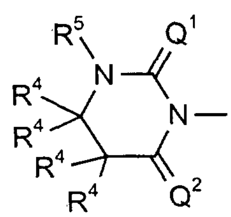
($Z^{21}$)

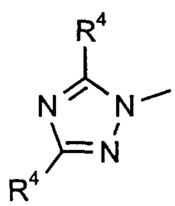
($Z^{22}$)

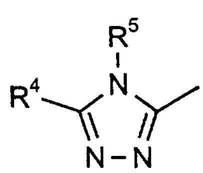
($Z^{23}$)

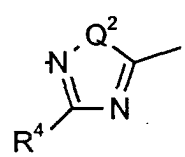
($Z^{24}$)

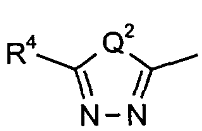
($Z^{25}$)

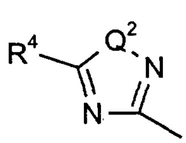
($Z^{26}$)

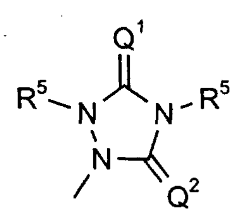
($Z^{27}$)

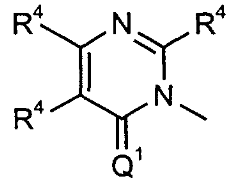

$(Z^{28})$

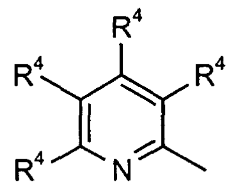

$(Z^{29})$

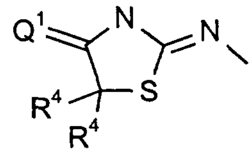

$(Z^{30})$

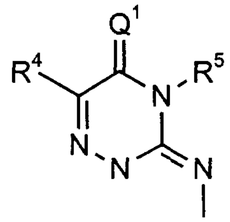

$(Z^{31})$

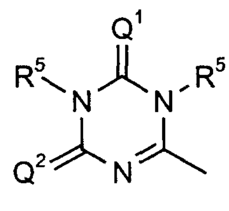

$(Z^{32})$

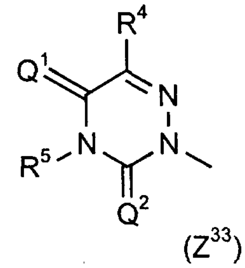

$(Z^{33})$

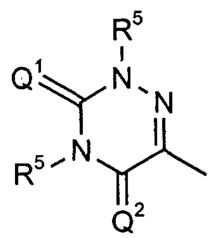

$(Z^{34})$

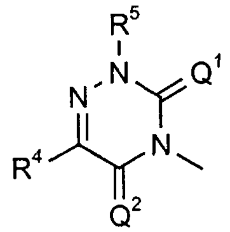

$(Z^{35})$

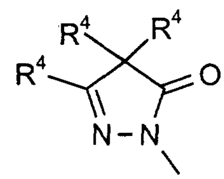

$(Z^{36})$

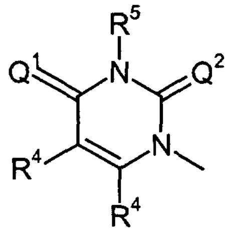

$(Z^{37})$

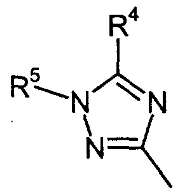

$(Z^{38})$

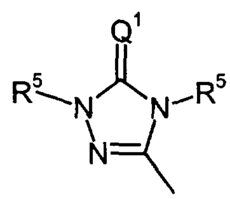

$(Z^{39})$

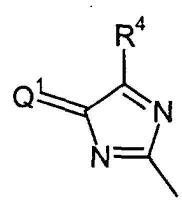

(Z^40)

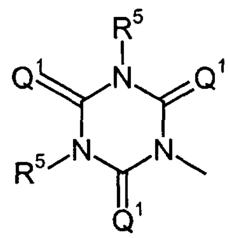

(Z^41)

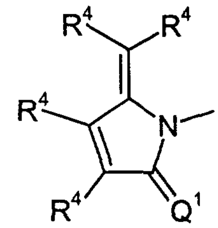

(Z^42)

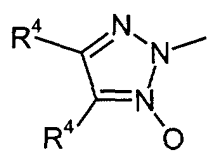

(Z^43)

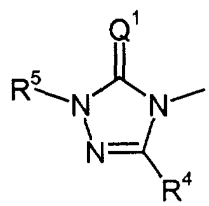

(Z^44)

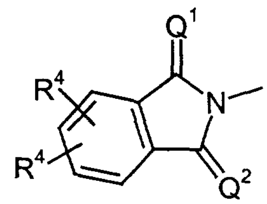

(Z^45)

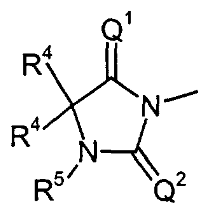

(Z^46)

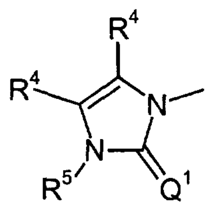

(Z^47)

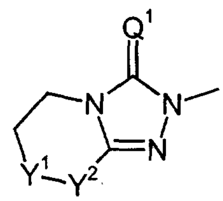

(Z^48)

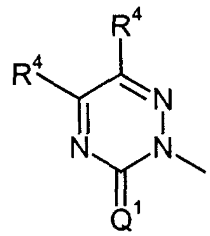

(Z^49)

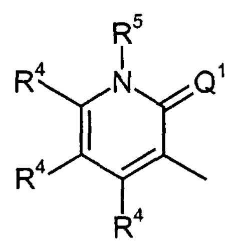

(Z^50)

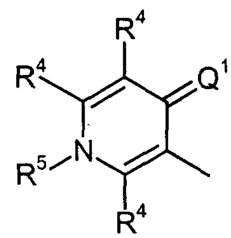

(Z^51)

wobei

$Q^1$ für O oder S steht,

$Q^2$ für O oder S steht,

$R^4$ für Wasserstoff, Amino, Nitro, Cyano, Carboxy, Carbamoyl, Thiocarbamoyl, Halogen, für gegebenenfalls durch Cyano, Halogen oder $C_1$-$C_4$-Alkoxy substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Cyano, Halogen oder $C_1$-$C_4$-Alkoxy substituiertes Alkoxy oder Alkoxycarbonyl mit jeweils 1 bis 6 Kohlenstoffatomen in den Alkylgruppen, für jeweils gegebenenfalls durch Halogen substituiertes Alkenyloxy oder Alkinyloxy mit jeweils 3 bis 6 Kohlenstoffatomen, für gegebenenfalls durch Cyano, Halogen oder $C_1$-$C_4$-Alkoxy substituiertes Alkylthio mit 1 bis 6 Kohlenstoffatomen, für jeweils gegebenenfalls durch Halogen substituiertes Alkenylthio oder Alkinylthio mit jeweils 3 bis 6 Kohlenstoffatomen, für Alkylamino oder Dialkylamino mit jeweils 1 bis 6 Kohlenstoffatomen in den Alkylgruppen, oder für jeweils gegebenenfalls durch Cyano, Halogen oder $C_1$-$C_4$-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil steht,

$R^5$ für Wasserstoff, Hydroxy, Amino, Cyano, für jeweils gegebenenfalls durch Cyano, Halogen oder $C_1$-$C_4$-Alkoxy substituiertes Alkyl, Alkoxy, Alkoxycarbonyl oder Alkylamino mit jeweils bis zu 6 Kohlenstoffatomen, für jeweils gegebenenfalls durch Halogen substituiertes Alkenyl oder Alkinyl mit jeweils bis zu 6 Kohlenstoffatomen, für jeweils gegebenenfalls durch Cyano, Halogen oder $C_1$-$C_4$-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Phenyl oder Phenyl-$C_1$-$C_4$-alkyl steht,

$Y^1$ für O, S, SO, $SO_2$, NH, N(Alkyl) oder Methylen steht und

$Y^2$ für eine Einfachbindung oder für O, S, SO, $SO_2$, NH oder N(Alkyl) steht,
wobei $Y^1$ und $Y^2$ in jedem Einzelfall verschieden sind.

$Z^2$ steht bevorzugt für die nachstehende Gruppierung

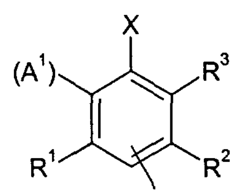

worin

$R^1$ für Wasserstoff, Nitro, Cyano oder Halogen steht,

$R^2$ für Nitro, Hydroxy, Cyano, Carbamoyl, Thiocarbamoyl oder für jeweils gegebenenfalls durch Halogen substituiertes Alkyl oder Alkoxy mit jeweils 1 bis 4 Kohlenstoffatomen steht, und

$R^3$ für Nitro, Hydroxy, Mercapto, Amino, Hydroxyamino, Cyano, Carboxy, Carbamoyl, Thiocarbamoyl, Halogen, für jeweils gegebenenfalls durch Cyano, Carboxy, Carbamoyl, Halogen, $C_1$-$C_4$-Alkoxy oder $C_1$-$C_4$-Alkoxy-carbonyl substituiertes Alkyl, Alkylcarbonyl, Alkoxy, Alkoxycarbonyl, Alkylthio, Alkylsulfinyl, Alkylsulfonyl oder Alkylamino mit jeweils 1 bis 6 Kohlenstoffatomen in den Alkylgruppen, für jeweils gegebenenfalls durch Halogen substituiertes Alkylsulfonylamino, N,N-Bis-alkylsulfonyl-amino, N-Alkylcarbonyl-N-alkylsulfonyl-amino mit jeweils 1 bis 4 Kohlenstoffatomen in den Alkylgruppen, für jeweils gegebenenfalls durch Cyano, Carboxy, Carbamoyl, Halogen oder $C_1$-$C_4$-Alkoxy-carbonyl substituiertes Alkenyl, Alkenyloxy, Alkenylthio, Alkenylamino, Alkinyl, Alkinyloxy, Alkinylthio, Alkinylamino mit jeweils bis zu 6 Kohlenstoffatomen in den Alkenyl- bzw. Alkinyl-gruppen, für jeweils gegebenenfalls durch Cyano, Carboxy, Carbamoyl, Halogen oder $C_1$-$C_4$-Alkyl substituiertes Cycloalkyl, Cycloalkyloxy, Cycloalkylthio, Cycloalkylamino, Cycloalkylsulfonylamino, Cycloalkylalkyl, Cycloalkylalkoxy, Cycloalkylalkylthio oder Cycloalkylalkylamino mit jeweils 3 bis 6 Kohlenstoffatomen in den Cycloalkylgruppen und gegebenenfalls 1 bis 4 Kohlenstoffatomen in den Alkylteilen, oder für jeweils gegebenenfalls durch Nitro, Cyano, Carboxy, Carbamoyl, Halogen, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkoxy-carbonyl substituiertes Aryl, Aryloxy, Arylthio, Arylamino, Arylalkyl, Arylalkoxy, Arylalkylthio, Arylalkylamino, N-Arylcarbonyl-N-alkylsulfonyl-amino mit jeweils 6 oder 10 Kohlenstoffatomen in den Arylgruppen und gegebenenfalls 1 bis 4 Kohlenstoffatomen im Alkylteil steht, und

X für Wasserstoff oder Halogen steht.

Besonders hervorgehoben wird weiterhin eine Substanz, welche die folgende Struktur aufweist:

$$Z^1\text{-}A^1\text{-}Z^2\text{-}$$

worin

$A^1$ für O steht,

$Z^1$ für eine der nachstehenden Gruppierungen steht

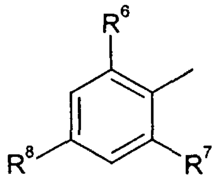

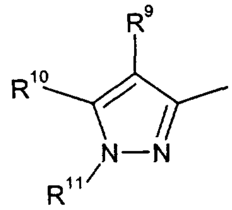

wobei

$R^6$ für Cyano oder Halogen steht,

$R^7$ für Wasserstoff oder Halogen steht,

$R^8$ für Cyano, Halogen, oder für Halogenalkyl oder Halogenalkoxy mit jeweils 1 bis 4 Kohlenstoffatomen steht,

$R^9$ für Wasserstoff oder Halogen steht,

$R^{10}$ für Halogenalkyl oder Halogenalkoxy mit jeweils 1 bis 4 Kohlenstoffatomen steht, und

$R^{11}$ für Alkyl mit 1 bis 4 Kohlenstoffatomen steht, und

$Z^2$ für die nachstehende Gruppierung steht

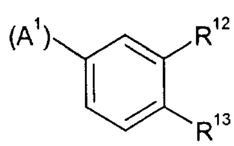

worin

$R^{12}$ für Carboxy oder für jeweils gegebenenfalls durch Cyano, Halogen oder $C_1$-$C_4$-Alkoxy substituiertes Alkoxy oder Alkoxycarbonyl mit jeweils bis zu 4 Kohlenstoffatomen steht und

$R^{13}$ für Cyano, Carbamoyl, Thiocarbamoyl, Halogen, oder für Halogenalkyl oder Halogenalkoxy mit jeweils 1 bis 4 Kohlenstoffatomen steht.

Die Substanz weist besonders bevorzugt die folgende allgemeine Formel auf:

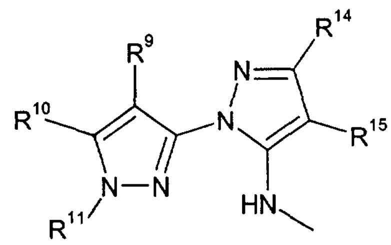

worin

$R^9$, $R^{10}$ und $R^{11}$ die oben angegebene Bedeutung haben,

$R^{14}$ für Wasserstoff, Halogen oder Alkyl mit 1 bis 4 Kohlenstoffatomen steht, und

$R^{15}$ für Nitro, Cyano, Carbamoyl, Thiocarbamoyl, oder für jeweils gegebenenfalls durch Cyano, Halogen oder $C_1$-$C_4$-Alkoxy substituiertes Alkyl oder Alkoxy mit jeweils 1 bis 4 Kohlenstoffatomen steht.

Mit einem Fluoreszenzfarbstoff markierte Substanzen wie sie vorstehend beschrieben sind, sind per se Gegenstand der vorliegenden Erfindung.

Als zu testende Substanzen, die in die erfindungsgemäßen Verfahren eingesetzt werden können, kommen beispielsweise organisch-chemische Moleküle, Naturstoffe und davon abgeleitete Verbindungen, oder Peptide in Frage. Vorzugsweise werden kleine organische-chemische Moleküle oder Gemische davon, die beispielsweise mit Hilfe der kombinatorischen Chemie synthetisiert wurden, eingesetzt. Es können auch Bestandteile aus Kulturbrühen, Zellextrakte oder konditionierte Medien eingesetzt werden.

Die fluoreszierende Substanz liegt im Testansatz vorzugsweise in einer Konzentration von weniger als 1 µM, besonders bevorzugt von weniger als 100 nM und ganz besonders bevorzugt von weniger als 10 nM vor.

Eine Vielzahl unterschiedlicher weiterer Reagenzien kann dem Testansatz zugesetzt werden. Dazu gehören verschiedene Reagenzien, wie z.B. Salze, Puffer, Proteine, wie z.B. Albumin oder Gamma-Globuline, Detergentien, Proteaseinhibitoren, Lösungsmittel usw. Zweck solcher Zusätze ist es, entweder direkt die Bindungsreaktion zu beeinflussen, sei es durch Erhöhung der Bindungsstärke, durch Verringerung der Bindungsstärke oder durch Erzeugung einer im Sinn des Testverfahrens zweckmäßig veränderten Kinetik der Bindung der fluoreszierenden Substanz bzw. der zu testenden Substanz oder des Gemisches aus zu testenden Substanzen an das Target-Protein PPO. Zweck dieser Zusätze kann aber auch beispielsweise eine Stabilisierung des Target-Proteins PPO sein, z.B. durch die Hemmung von im Testansatz möglicherweise vorhandenen Proteasen, durch die positive Beeinflussung der Löslichkeit der einzelnen Komponenten im Testansatz, oder durch die Verhinderung von möglicherweise auftretenden Quencheffekten im Testansatz.

Der Testansatz wird unter Bedingungen inkubiert, bei denen die fluoreszierende Substanz an das Target-Enzym PPO binden kann.

In einer Ausführungsform der Erfindung befinden sich alle Komponenten der Mischung in Lösung bzw. in Suspension, wenn der Testansatz unlösliche Komponenten, beispielsweise schlecht lösliche Testsubstanzen enthält, bzw. wenn das Target-Protein PPO als Rohextrakt eingesetzt wird.

Die Bestandteile der Mischung im Testansatz können in jeder beliebigen Reihenfolge zusammengegeben werden, sofern unter solchen Bedingungen eine geeignete molekulare Wechselwirkung der Komponenten der Mischung, insbesondere eine Bindung der fluoreszierenden Substanz an das Target-Protein, möglich ist.

Die Inkubation erfolgt unter geeigneter Temperierung. Geeignet sind beispielsweise Inkubationstemperaturen zwischen 4°C und 60°C, vorzugsweise zwischen 15°C und 45°C und besonders bevorzugt zwischen 20°C und 40°C. Während der Inkubation kann der Ansatz geschüttelt werden.

Der Test wird in verschiedenen gängigen Formaten in Mikrotiterplatten bzw. auf Oberflächen bzw. in Küvetten- oder Kapillarsystemen durchgeführt.

Gegenstand der vorliegenden Erfindung ist auch ein Test-System, umfassend

a) Behältnisse mit Mischungen, wie vorstehend definiert,

b) eine Vorrichtung zum Bestrahlen der Mischungen mit linear polarisiertem Licht einer Wellenlänge, das die fluoreszierende Substanz zur Emission von Licht anregt, und

c) eine Vorrichtung zum Messen der Fluoreszenzpolarisationswerte oder der Anisotropiewerte des emittierten Lichts.

Weiterhin sind Substanzen, die mit dem Enzym Protoporphyrinogen-Oxidase wechselwirken und durch die erfindungsgemäßen Verfahren identifiziert wurden, Gegenstand der vorliegenden Erfindung. Solche Substanzen stellen potenzielle Herbizide dar.

Die erfindungsgemäßen, mit einem Fluoreszenzfarbstoff markierten Substanzen können auch in Testverfahren eingesetzt werden, die auf Fluoreszenz-Korrelations-Spektroskopie basieren (vgl. Auer et al. 1998, Fluorescence correlations spectroscopy: lead discovery by miniaturized HTS, Drug Discovery Today 3, 457).

Im Folgenden wird die vorliegende Erfindung an Hand von Beispielen weiter erläutert.

## Beispiele:

### Beispiel 1

Synthese von Ligand 2: 2-(2-Amino-ethoxy)-4-(4-brom-5-difluormethoxy-1-methyl-1H-pyrazol-3-yl)-5-fluor-benzonitril

Zu einer Lösung von 1,34 g (22 mMol) Ethanolamin in 30 ml wasserfreiem Acetonitril gibt man 0,31 g (7,7 mMol) 60 %iges Natriumhydrid in Paraffinöl. Die Mischung wird 30 Minuten bei Raumtemperatur gerührt und danach mit einer Lösung von 2,0 g (5,5 mMol) 4-(4-Brom-5-difluormethoxy-1-methyl-lH-pyrazol-3-yl)-2,5-difluor-benzonitril versetzt. Man erwärmt die Reaktionslösung unter Rühren sechs Stunden auf 50°C, trägt danach auf 100 ml Eiswasser aus und extrahiert die entstandene Emulsion mit Dichlormethan. Die organische Phase wird nacheinander mit Wasser und gesättigter Kochsalzlösung gewaschen, über Magnesiumsulfat getrocknet und im Vakuum vom Lösungsmittel befreit. Der ölige Rückstand wird nacheinander mit Wasser und Diethylether verrührt, der Feststoff abgesaugt und bei 40°C im Vakuum getrocknet.

Man erhält 0,87 g (40 % der Theorie) 2-(2-Amino-ethoxy)-4-(4-brom-5-difluormethoxy-1-methyl-1H-pyrazol-3-yl)-5-fluor-benzonitril. Schmp.: 101°C

### Beispiel 2

Konjugation von Ligand 2 mit fluorophoren Gruppen

Herstellung von Tracer A: Konjugation von Ligand 2 mit Fluorescein 5-EX, Succinimidylester

Lösung 1: 5,66 mg Ligand 2 (= 2-(2-Aminoethoxy)-4-(4-brom-5-difluormethoxy-1-methyl-1H-pyrazol-3-yl)-5-fluor-benzonitril) werden gelöst in 1,415 ml DMSO.

Lösung 2: 10 mg Fluorescein-5-EX,SE (Fa. Molecular Probes, Bestell-Nr. F-6130) werden gelöst in 0,25 ml DMSO.

Lösung 1 und 2 werden zusammengegeben und im Dunkeln bei Raumtemperatur 1 Stunde inkubiert.

Anschliessend wird das Reaktionsprodukt durch semipräparative HPLC unter Verwendung der folgenden Instrumentenparameter rein dargestellt:
HPLC-Gerät: Äkta Explorer (Fa. Amersham Pharmacia)

Stationäre Phase: NUCLEOSIL 100-7 C 18 von Fa. Macherey & Nagel (7 µm; 250 x 10 mm; Säulenvolumen ca.19,63 ml). Diese Säule wurde für die semipräparative Reinigung des Produkts (Tracer A) eingesetzt. In jedem Lauf wurden 250 µl Probe aufgetragen und fraktioniert.

Elution: Die Säule wurde eluiert mit einem Gradienten aus Fließmittel A (0,5% Ameisensäure p.A.) und Fließmittel B (Acetonitril ); die Flussrate betrug 6 ml/min.

Der Gradient wurde wie folgt durchgeführt:
Start: 50% A / 50% B; in 5,5 Säulenvolumen auf 35% A / *65%* B.

Detektion: Das Reaktionsprodukt (Tracer A) wurde mittels UV-Messung bei 495 nm detektiert. Die Retentionszeit betrug 9,0 min.

Die ausgewählten Fraktionen wurden vereinigt, am Rotationsverdampfer unter Vakuum bei 22°C einrotiert und gefriergetrocknet. Die Zuordnung der Strukturen erfolgte mit Hilfe der Massenspektrometrie.

Insgesamt wurden 2,8 mg des als Tracer A bezeichneten Produkts gewonnen.

Molmasse = 878; Summenformel: $C_{39}H_{29}BrF_3N_5O_9S$

**Beispiel 3**

Herstellung von Tracer B: Konjugation von Ligand 2 mit 5-Carboxyfluorescein, Succinimidylester

Lösung 1: 1 mg Ligand 2 (2-(2-Aminoethoxy)-4-(4-brom-5-difluormethoxy-1-methyl-1H-pyrazol-3-yl)-5-fluorbenzonitril) wurde gelöst in 1 ml PBS/DMSO (60 : 40).
Herstellung von PBS (Phospate Buffered Saline): 8 g NaCl, 0,2 g KCl, 144 g $Na_2HPO_4$ und 0,24 g $KH_2PO_4$ wurden in 800 ml destilliertem Wasser gelöst, ein pH-Wert von 7,4 eingestellt und mit destilliertem Wasser auf 1 1 aufgefüllt.
Lösung 2: 4 mg 5-FAM,SE ( 5-Carboxyfluorescein, Succinimidylester, Single Isomer, Fa. Molecular Probes, Bestell-Nr. C-2210) wurden in 0,4 ml Acetonitril/DMSO (1:1) gelöst. Für den Labeling-Ansatz wurde lml Lösung 1 mit 0,2 ml Lösung 2 versetzt und im Dunkeln bei Raumtemperatur 5 Stunden lang inkubiert.
Die Auftrennung des Produkts und die Reinigung von Tracer B erfolgte unter den gleichen Bedingungen wie im Beispiel 2 mit Hilfe der HPLC unter Verwendung eines Äkta-HPLC-Systems. Die Retentionszeit von Tracer B war 10,5 min.
Die Ausbeute an Produkt (Tracer B) betrug 1,1 mg. Die Analyse mittels Massenspektrometrie ergab eine Molmasse von 763 g/mol.
Summenformel von Tracer B: $C_{35}H_{22}BrF_3N_4O_8$

**Beispiel 4**

Herstellung eines PPO-haltigen Proteinrohextrakts aus Gerste

Gerste wurde in Vermiculit ausgesät, 6 Tage im Dunkeln bei Raumtemperatur angezogen, mit jeweils 2 Stunden Belichtung.
5 g Blätter von den erhaltenen Gerstenpflanzen wurden zerkleinert und mit 20 ml Aufarbeitungspuffer bei 4°C homogenisiert.
Das Homogenat wurde durch Nylongaze (Maschenweite 0,1 mm) abfiltriert. Durch Zentrifugation wurde aus dem Filtrat die Plastidenfraktion gewonnen; die Zentrifugation wurde bei 2500 Umdrehungen pro Minute bei 4°C in einer Tischzentrifuge durchgeführt (Fa. Eppendorf, 5810 R, Rotor Typ A-4-62). Die Dauer der Zentrifugation betrug 10 Minuten. Nach der Zentrifugation wurde der Überstand dekantiert und das Pellet in 1 ml Aufarbeitungspuffer resuspendiert. Der Aufarbeitungspuffer hatte folgende Zusammensetzung: 350 mM Saccharose, 10 mM $KH_2PO_4$, 10 mM Tris.HCl, 1 mM EDTA, 1 mM $MgCl_2$, 10 mM 2-Mercaptoethanol, 0,1 % BSA, mit HCl und NaOH auf pH 7,4 eingestellt.
Die so hergestellte PPO-haltige Etioplastenfraktion aus Gerste besaß einen Proteingehalt von 2,2 mg/ml. Der Proteingehalt wurde mit Hilfe des Bio-Rad Proteinassays bestimmt (Bradford, 1976; vgl. Anleitung der Fa. Bio-Rad), die Durchführung erfolgte als Microassay. Jeweils 0,8 ml einer Verdünnungsreihe des Proteinextrakts wurde mit 0,2 ml Dye Reagent Concentrate versetzt und mittels Vortex durchmischt. Nach 10 Minuten wurde die Optische Dichte bei 595 nm im Photometer (Fa. Beckmann spektrophotometer Typ DU 640) gemessen und aus einer Proteinstandardkurve der entsprechende Wert abgelesen; als Proteinstandard wurde Rinderserumalbumin (BSA) verwendet.

**Beispiel 5**

Bindung der Tracer A und B an das Target-Enzym PPO

Zum Nachweis der Bindung an das Targetprotein PPO wurden der Tracer A bzw. der Tracer B in DMSO gelöst; die Konzentration dieser Stammlösungen betrug 1 µM. In die Löcher einer schwarzen 96 Loch-Mikrotiterplatte (Fa. Greiner; FIA-Platten F-Form schwarz) wurden pipettiert:

1) 2,5 µl einer Verdünnung der jeweiligen Tracer-Stammlösung; die Endkonzentration der jeweiligen Tracer betrug je nach Ansatz zwischen 5 x $10^{-7}$ und 5 x $10^{-10}$ mol/l;

2) 97,5 µl PPO-Lösung aus Gerste (Endkonzentration an Protein: 1000 µg/ml im Ansatz).

Anschließend wurde der Ansatz 10 Minuten bei Raumtemperatur (ca. 22°C) unter Schütteln auf einem Eppendorf-Thermomixer (bei einer Schüttelbewegung von 650 Umdrehungen pro Minute) inkubiert. Der DMSO-Gehalt im Testgemisch betrug 2,5 %.
Danach wurde die Polarisation des Ansatzes im Plattenreader Ultra (Fa. Tecan) gemessen. Alternativ kann die Anisotropie des Gemischs bestimmt werden.

Die Bedingungen bei der Messung der Polarisation waren wie folgt:

Anregungswellenlänge ($\lambda_{ex}$): 485 nm
Emissionswellenlänge ($\lambda_{em}$): 530 nm
Anzahl der Lichtblitze pro Loch: 30

Der Gain des Geräts wurde manuell auf den Wert 60 eingestellt.
Als Referenzen dienten Standards der Fa. PanVera (High und Low Polarization Standards).

**Beispiel 6**

Bindung von potenziell herbizid aktiven Verbindungen an PPO: Test der Substanzen 5-Amino-1-(2,6-dichlor-4-trifluoromethyl-phenyl)-4-nitro-1H-pyrazol und 2-(2-Aminoethoxy)-4-(4-brom-5-difluormethoxy-1-methyl-1H-pyrazol-3-yl)-5-fluorbenzonitril

Die Proteinlösung (vgl. Beispiel 5, Enzymextrakt aus Gerste; Proteinkonzentration 2,2 mg/ml) wurde mit Kaliumphosphatpuffer pH 8,0 auf eine Proteinkonzentration von 1 mg/ml verdünnt.
In die Löcher einer schwarzen 96 Loch-Mikrotiterplatte (Fa. Greiner; FIA-Platten F-Form schwarz) wurden pipettiert:

1) 10,5 µl der Tracer-Lösung (250 nM Tracer A in DMSO); die Endkonzentration des Tracers betrug 1,25 nM;

2) 2,5 µl einer Lösung der Testsubstanzen in DMSO;

3) 97 ml PPO-Lösung aus Gerste.

Anschließend wurde der Ansatz 10 Minuten bei Raumtemperatur (ca. 22°C) unter Schütteln auf einem Eppendorf-Thermomixer (bei einer Schüttelbewegung von 650 Umdrehungen pro Minute) inkubiert. Der DMSO-Gehalt im Testgemisch betrug 3%.
Danach wurde die Polarisation des Ansatzes im Plattenreader Ultra (Fa. Tecan) gemessen. Alternativ wurde die Anisotropie des Gemischs bestimmt.
Die Bedingungen bei der Messung der Polarisation waren wie folgt:

Anregungswellenlänge ($\lambda_{ex}$): 485 nm
Emissionswellenlänge ($\lambda_{em}$): 535 nm
Anzahl der Lichtblitze pro Loch: 30

Der Gain des Geräts wurde manuell auf den Wert 75 eingestellt.
Als Referenzen dienten Standards der Fa. PanVera (High und Low Polarization Standards). Die Angabe der Polarisationswerte erfolgte, wie bei Polarisationsmessungen üblich, in der Einheit mP.
Die Versuchsauswertung erfolgte mit Hilfe der Software Graph Pad Prism (GraphPadSoftware, Inc.).
Folgende $IC_{50}$-Werte wurden erhalten:
Ligand 1 ( = 5-Amino-1-[2,6-dichlor-4-(trifluormethyl)phenyl]-4-nitro-1H-pyrazol): $IC_{50}$ = 1,72 x $10^{-8}$ mol/l
Ligand 2 ( = 2(2-Aminoethoxy)-4[4-brom-5-(difluormethoxy)-1-methyl-1H-pyrazol-3-yl]-5-fluor-benzonitril): $IC_{50}$ = 3,94 x $10^{-9}$ mol/l.

**Patentansprüche**

**1.** Verfahren zum Auffinden von Substanzen, die mit dem Enzym Protoporphyrinogen-Oxidase (PPO) wechselwirken, umfassend die folgenden Schritte:

a) Herstellen von Mischungen, die (i) PPO, (ii) eine bei geeigneter Bestrahlung fluoreszierende Substanz, die mit PPO in Wechselwirkung treten kann, und (iii) eine zu testende Substanz oder ein Gemisch aus zu testenden Substanzen in unterschiedlichen Konzentrationen umfassen,

b) Bestrahlen der Mischungen mit linear polarisiertem Licht einer geeigneten Wellenlänge, das die fluoreszierende Substanz zur Emission von Licht anregt, und

c) Messen der Fluoreszenzpolarisationswerte oder der Anisotropiewerte des emittierten Lichts,

wobei eine Verringerung des Fluoreszenzpolarisationswertes bei steigender Konzentration der zu testenden Substanz oder des Gemisches aus zu testenden Substanzen eine Wechselwirkung einer oder mehrerer zu testenden Substanzen mit der PPO anzeigt.

**2.** Verfahren zum Testen, ob eine Substanz mit dem Enzym Protoporphyrinogen-Oxidase (PPO) wechselwirkt, umfassend die folgenden Schritte:

a) Herstellen von Mischungen, die (i) PPO und (ii) eine bei geeigneter Bestrahlung fluoreszierende Substanz in unterschiedlichen Konzentrationen umfassen,

b) Bestrahlen der Mischungen mit linear polarisiertem Licht einer geeigneten Wellenlänge, das die fluoreszierende Substanz zur Emission von Licht anregt,

c) Messen der Fluoreszenzpolarisationswerte oder der Anisotropiewerte des emittierten Lichts,

wobei eine Erhöhung des Fluoreszenzpolarisationswertes bei fallender Konzentration der fluoreszierenden Substanz eine Wechselwirkung mit der PPO anzeigt.

**3.** Verfahren gemäß Anspruch 1 oder 2, **dadurch gekennzeichnet, dass** die PPO eine pflanzliche PPO ist.

**4.** Verfahren gemäß einem der Ansprüche 1 bis 3, **dadurch gekennzeichnet, dass** ein PPO enthaltender Pflanzenzellextrakt eingesetzt wird.

**5.** Verfahren gemäß einem der Ansprüche 1 bis 3, **dadurch gekennzeichnet, dass** aus Pflanzenzellextrakten biochemisch gereinigte PPO eingesetzt wird.

**6.** Verfahren gemäß einem der Ansprüche 1 bis 3, **dadurch gekennzeichnet, dass** mittels gentechnischer Verfahren hergestellte PPO eingesetzt wird.

**7.** Verfahren gemäß einem der Ansprüche 1 bis 6, **dadurch gekennzeichnet, dass** es sich bei der fluoreszierenden Substanz um eine mit einem Fluoreszenzfarbstoff markierte Substanz handelt.

**8.** Verfahren gemäß Anspruch 7, **dadurch gekennzeichnet, dass** es sich bei dem Fluoreszenzfarbstoff um Fluorescein oder ein Fluorescein-Derivat handelt.

**9.** Verfahren gemäß einem der Ansprüche 1 bis 8, **dadurch gekennzeichnet, dass** es sich bei der Substanz, die mit PPO wechselwirken kann, um Liganden der PPO, natürliche PPO-Substrate, natürliche Produkte der PPO-Enzymreaktion oder um herbizid wirksame PPO-Hemmstoffe handelt.

**10.** Verfahren gemäß einem der Ansprüche 1 bis 9, **dadurch gekennzeichnet, dass** die fluoreszierende Substanz die folgende Struktur aufweist:

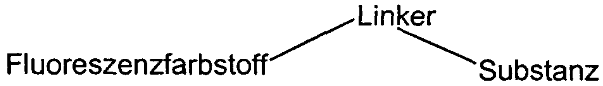

wobei
"Linker"
für eine jeweils geradkettige oder verzweigte, jeweils gesättigte oder ungesättigte, jeweils gegebenenfalls substituierte, jeweils an einem Ende mit der Substanz und am anderen Ende mit dem Fluoreszenzfarbstoff verknüpfte Kohlenwasserstoffkette steht, welche jeweils am Anfang oder am Ende oder innerhalb der Kette eine oder mehrere der nachstehenden Hetero-Komponenten enthalten kann:

$$Q^1, CQ^2, CQ^2\text{-}Q^1, Q^1\text{-}CQ^2, Q^1\text{-}CQ^2\text{-}Q^1, SO, SO_2$$

wobei jeweils $Q^1$ und $Q^2$ für O, S oder NH stehen, oder für eine jeweils gesättigte oder ungesättigte, jeweils gegebenenfalls substituierte an einem Ende mit der Substanz und am anderen Ende mit dem Fluoreszenzfarbstoff verknüpfte, carbocyclische oder heterocyclische Gruppierung steht, und
"Substanz"
für eine Substanz steht, welche mit PPO in Wechselwirkung treten kann.

**11.** Mit einem Fluoreszenzfarbstoff markierte Substanz, **gekennzeichnet durch** die folgende Struktur:

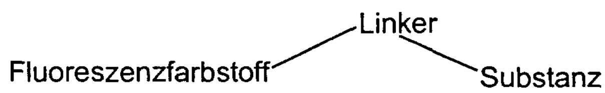


wobei
"Linker"
für eine jeweils geradkettige oder verzweigte, jeweils gesättigte oder ungesättigte, jeweils gegebenenfalls substituierte, jeweils an einem Ende mit der Substanz und am anderen Ende mit dem Fluoreszenzfarbstoff verknüpfte Kohlenwasserstoffkette steht, welche jeweils am Anfang oder am Ende oder innerhalb der Kette eine oder mehrere der nachstehenden Hetero-Komponenten enthalten kann:

$$Q^1, CQ^2, CQ^2\text{-}Q^1, Q^1\text{-}CQ^2, Q^1\text{-}CQ^2\text{-}Q^1, SO, SO^2$$

wobei jeweils $Q^1$ und $Q^2$ für O, S oder NH stehen, oder für eine jeweils gesättigte oder ungesättigte, jeweils gegebenenfalls substituierte an einem Ende mit der Substanz und am anderen Ende mit dem Fluoreszenzfarbstoff verknüpfte, carbocyclische oder heterocyclische Gruppierung steht, und
"Substanz"
für eine Substanz steht, welche mit PPO in Wechselwirkung treten kann.

**12.** Substanz gemäß Anspruch 11, **dadurch gekennzeichnet, dass**
"Fluoreszenzfarbstoff"
für eine Farbstoff-Gruppierung steht, welche durch die nachstehende Formel **gekennzeichnet** ist,

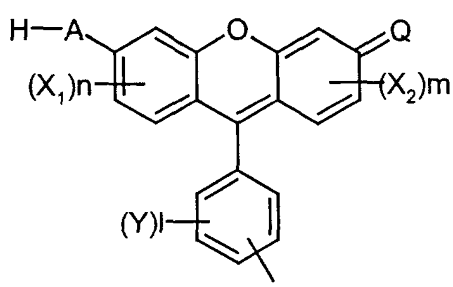

worin

A für O oder NH steht,

Q für O, S oder NH steht,

$X^1$ für Wasserstoff, Halogen, Nitro, Hydroxy, Carboxy (COOH), Sulfo ($SO_3H$) oder Alkyl steht,

$X^2$ für Wasserstoff, Halogen, Nitro, Hydroxy, Carboxy (COOH), Sulfo ($SO_3H$) oder Alkyl steht,

Y für Halogen, Alkyl, Carboxy (COOH) oder Sulfo ($SO_3H$) steht,

1 für die Indizes 0 bis 4 steht, und m, n für die Indizes 0 bis 3 steht.

**13.** Substanz gemäß Anspruch 12, **dadurch gekennzeichnet, dass**
A für O steht.

**14.** Substanz gemäß Anspruch 12 oder 13, **dadurch gekennzeichnet, dass**
Q für O steht.

**15.** Substanz gemäß einem der Ansprüche 12 bis 14, **dadurch gekennzeichnet, dass**

$X^1$ für Wasserstoff, Fluor, Chlor, Brom oder Alkyl mit 1 bis 4 Kohlenstoffatomen steht, und

$X^2$ für Wasserstoff, Fluor, Chlor, Brom oder Alkyl mit 1 bis 4 Kohlenstoffatomen steht.

**16.** Substanz gemäß einem der Ansprüche 12 bis 15, **dadurch gekennzeichnet, dass**

$X^1$ für Wasserstoff, Fluor, Chlor, Methyl oder Ethyl steht, und

$X^2$ für Wasserstoff, Fluor, Chlor, Methyl oder Ethyl steht.

**17.** Substanz gemäß einem der Ansprüche 11 bis 16, **dadurch gekennzeichnet, dass** die Kohlenwasserstoffkette des Linkers bis zu 10 Kohlenstoffatome aufweist.

**18.** Substanz gemäß einem der Ansprüche 11 bis 17, **dadurch gekennzeichnet, dass** die carbocyclische oder heterocyclische Gruppierung des Linkers bis zu 10 Kohlenstoffatome und gegebenenfalls bis zu 5 Stickstoffatome und gegebenenfalls 1 oder 2 Sauerstoff- oder Schwefelatome aufweist.

**19.** Substanz gemäß einem der Ansprüche 11 bis 18, **dadurch gekennzeichnet, dass** die Substanz die folgende Struktur aufweist:

$$Z^1\text{-}A^1\text{-}Z^2\text{-}$$

wobei

$A^1$ für eine Einfachbindung, O oder S steht,

$Z^1$ für eine carbocyclische oder heterocyclische Gruppierung mit jeweils bis zu 10 Kohlenstoffatomen und gegebenenfalls bis zu 5 Stickstoffatomen und gegebenenfalls 1 oder 2 Sauerstoff- oder Schwefelatomen steht, und

$Z^2$ für eine carbocyclische oder heterocyclische Gruppierung mit jeweils bis zu 10 Kohlenstoffatomen und gegebenenfalls bis zu 5 Stickstoffatomen und gegebenenfalls 1 oder 2 Sauerstoff- oder Schwefelatomen steht.

**20.** Substanz gemäß Anspruch 19, **dadurch gekennzeichnet, dass**

$A^1$ für eine Einfachbindung steht,

$Z^1$ für eine der nachstehenden Gruppierungen steht

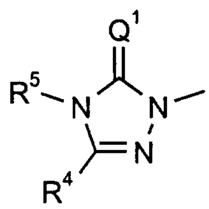
(Z1)

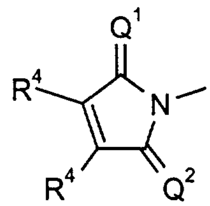
(Z2)

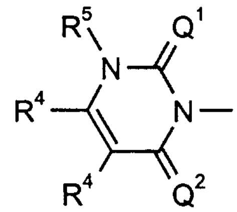
(Z3)

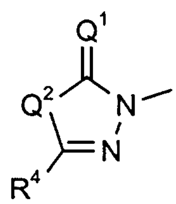
(Z4)

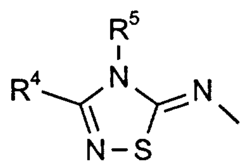
(Z5)

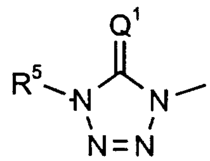
(Z6)

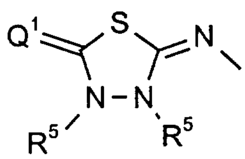
(Z7)

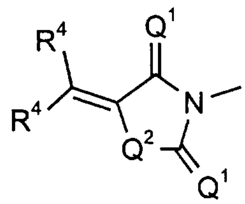
(Z8)

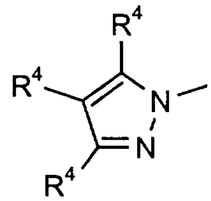
(Z9)

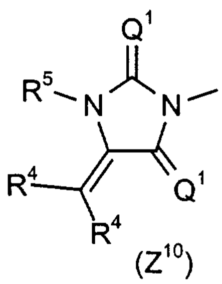
(Z10)

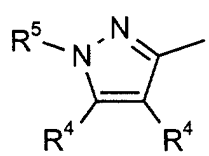
(Z11)

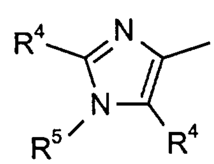
(Z12)

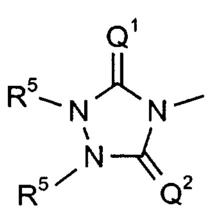
(Z13)

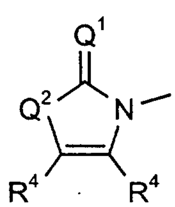
(Z14)

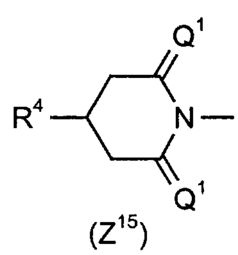
(Z15)

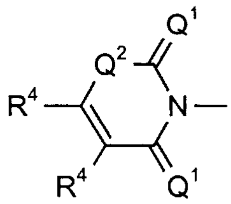

($Z^{16}$)

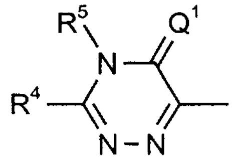

($Z^{17}$)

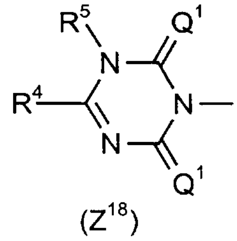

($Z^{18}$)

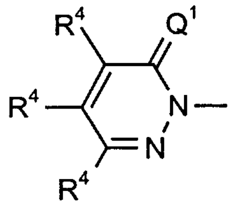

($Z^{19}$)

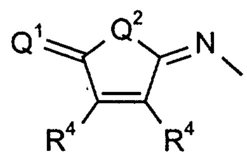

($Z^{20}$)

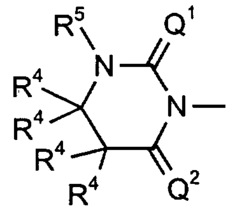

($Z^{21}$)

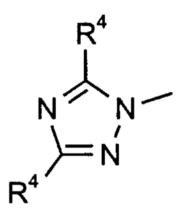

($Z^{22}$)

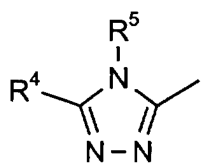

($Z^{23}$)

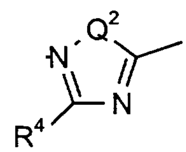

($Z^{24}$)

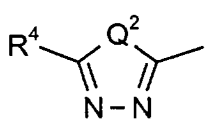

($Z^{25}$)

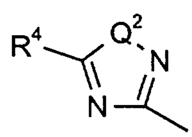

($Z^{26}$)

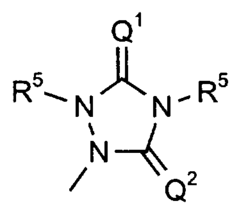

($Z^{27}$)

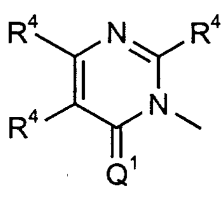

($Z^{28}$)

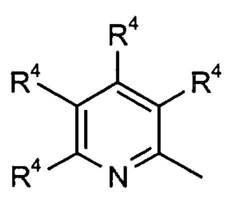

($Z^{29}$)

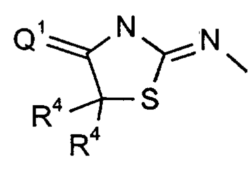

($Z^{30}$)

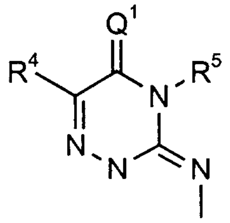
(Z31)

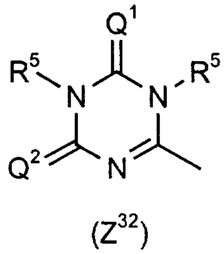
(Z32)

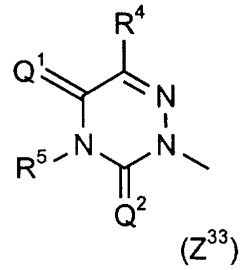
(Z33)

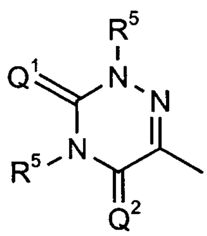
(Z34)

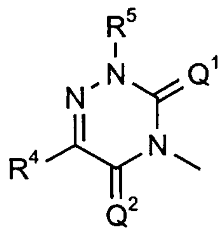
(Z35)

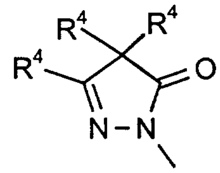
(Z36)

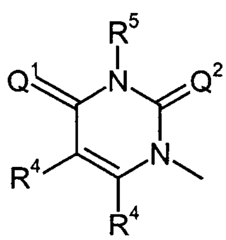
(Z37)

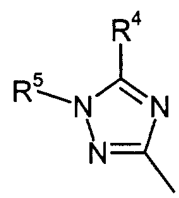
(Z38)

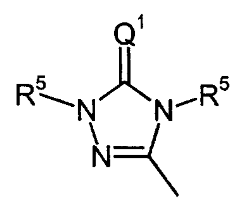
(Z39)

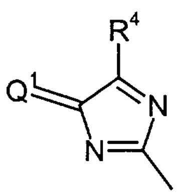
(Z40)

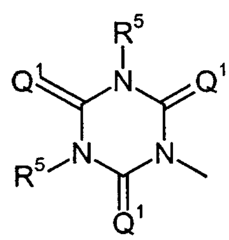
(Z41)

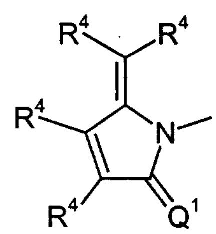
(Z42)

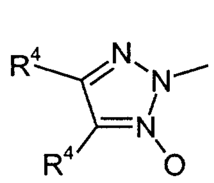 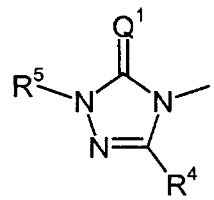 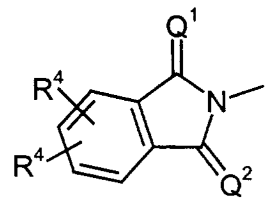

($Z^{43}$) ($Z^{44}$) ($Z^{45}$)

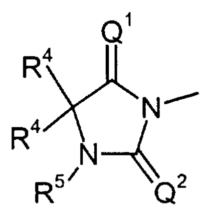 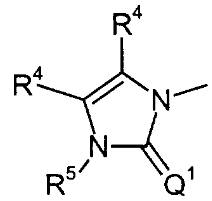 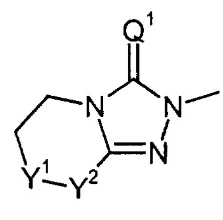

($Z^{46}$) ($Z^{47}$) ($Z^{48}$)

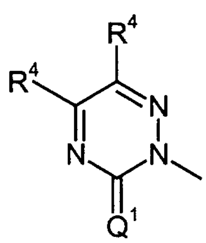 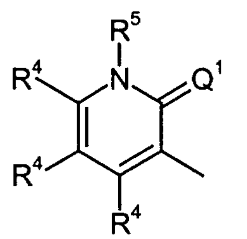 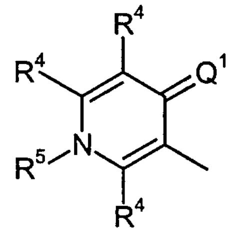

($Z^{49}$) ($Z^{50}$) ($Z^{51}$)

wobei

$Q^1$ für O oder S steht,

$Q^2$ für O oder S steht,

$R^4$ für Wasserstoff, Amino, Nitro, Cyano, Carboxy, Carbamoyl, Thiocarbamoyl, Halogen, für gegebenenfalls durch Cyano, Halogen oder $C_1$-$C_4$-Alkoxy substituiertes Alkyl mit 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Cyano, Halogen oder $C_1$-$C_4$-Alkoxy substituiertes Alkoxy oder Alkoxycarbonyl mit jeweils 1 bis 6 Kohlenstoffatomen in den Alkylgruppen, für jeweils gegebenenfalls durch Halogen substituiertes Alkenyloxy oder Alkinyloxy mit jeweils 3 bis 6 Kohlenstoffatomen, für gegebenenfalls durch Cyano, Halogen oder $C_1$-$C_4$-Alkoxy substituiertes Alkylthio mit 1 bis 6 Kohlenstoffatomen, für jeweils gegebenenfalls durch Halogen substituiertes Alkenylthio oder Alkinylthio mit jeweils 3 bis 6 Kohlenstoffatomen, für Alkylamino oder Dialkylamino mit jeweils 1 bis 6 Kohlenstoffatomen in den Alkylgruppen, oder für jeweils gegebenenfalls durch Cyano, Halogen oder $C_1$-$C_4$-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil steht,

$R^5$ für Wasserstoff, Hydroxy, Amino, Cyano, für jeweils gegebenenfalls durch Cyano, Halogen oder $C_1$-$C_4$-Alkoxy substituiertes Alkyl, Alkoxy, Alkoxycarbonyl oder Alkylamino mit jeweils bis zu 6 Kohlenstoffatomen,

für jeweils gegebenenfalls durch Halogen substituiertes Alkenyl oder Alkinyl mit jeweils bis zu 6 Kohlenstoffatomen, für jeweils gegebenenfalls durch Cyano, Halogen oder $C_1$-$C_4$-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Phenyl oder Phenyl$C_1$-$C_4$-alkyl steht,

$Y^1$ für O, S, SO, $SO_2$, NH, N(Alkyl) oder Methylen steht und

$Y^2$ für eine Einfachbindung oder für O, S, SO, $SO_2$, NH oder N(Alkyl) steht,
wobei $Y^1$ und $Y^2$ in jedem Einzelfall verschieden sind,
und

$Z^2$ für die nachstehende Gruppierung steht

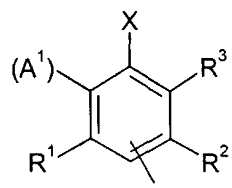

worin

$R^1$ für Wasserstoff, Nitro, Cyano oder Halogen steht,

$R^2$ für Nitro, Hydroxy, Cyano, Carbamoyl, Thiocarbamoyl oder für jeweils gegebenenfalls durch Halogen substituiertes Alkyl oder Alkoxy mit jeweils 1 bis 4 Kohlenstoffatomen steht, und

$R^3$ für Nitro, Hydroxy, Mercapto, Amino, Hydroxyamino, Cyano, Carboxy, Carbamoyl, Thiocarbamoyl, Halogen, für jeweils gegebenenfalls durch Cyano, Carboxy, Carbamoyl, Halogen, $C_1$-$C_4$-Alkoxy oder $C_1$-$C_4$-Alkoxy-carbonyl substituiertes Alkyl, Alkylcarbonyl, Alkoxy, Alkoxycarbonyl, Alkylthio, Alkylsulfinyl, Alkylsulfonyl oder Alkylamino mit jeweils 1 bis 6 Kohlenstoffatomen in den Alkylgruppen, für jeweils gegebenenfalls durch Halogen substituiertes Alkylsulfonylamino, N,N-Bis-alkylsulfonyl-amino, N-Alkylcarbonyl-N-alkylsulfonyl-amino mit jeweils 1 bis 4 Kohlenstoffatomen in den Alkylgruppen, für jeweils gegebenenfalls durch Cyano, Carboxy, Carbamoyl, Halogen oder $C_1$-$C_4$-Alkoxy-carbonyl substituiertes Alkenyl, Alkenyloxy, Alkenylthio, Alkenylamino, Alkinyl, Alkinyloxy, Alkinylthio, Alkinylamino mit jeweils bis zu 6 Kohlenstoffatomen in den Alkenyl- bzw. Alkinyl-gruppen, für jeweils gegebenenfalls durch Cyano, Carboxy, Carbamoyl, Halogen oder $C_1$-$C_4$-Alkyl substituiertes Cycloalkyl, Cycloalkyloxy, Cycloalkylthio, Cycloalkylamino, Cycloalkylsulfonylamino, Cycloalkylalkyl, Cycloalkylalkoxy, Cycloalkylalkylthio oder Cycloalkylalkylamino mit jeweils 3 bis 6 Kohlenstoffatomen in den Cycloalkylgruppen und gegebenenfalls 1 bis 4 Kohlenstoffatomen in den Alkylteilen, oder für jeweils gegebenenfalls durch Nitro, Cyano, Carboxy, Carbamoyl, Halogen, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkoxycarbonyl substituiertes Aryl, Aryloxy, Arylthio, Arylamino, Arylalkyl, Arylalkoxy, Arylalkylthio, Arylalkylamino, N-Arylcarbonyl-N-alkylsulfonyl-amino mit jeweils 6 oder 10 Kohlenstoffatomen in den Arylgruppen und gegebenenfalls 1 bis 4 Kohlenstoffatomen im Alkylteil steht, und

X für Wasserstoff oder Halogen steht.

**21.** Substanz gemäß Anspruch 19, **dadurch gekennzeichnet, dass**

$A^1$ für O steht,

$Z^1$ für eine der nachstehenden Gruppierungen steht

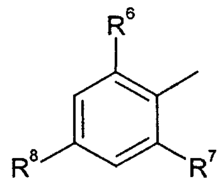 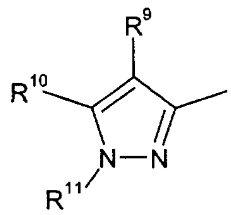

wobei

$R^6$ für Cyano oder Halogen steht,

$R^7$ für Wasserstoff oder Halogen steht,

$R^8$ für Cyano, Halogen, oder für Halogenalkyl oder Halogenalkoxy mit jeweils 1 bis 4 Kohlenstoffatomen steht,

$R^9$ für Wasserstoff oder Halogen steht,

$R^{10}$ für Halogenalkyl oder Halogenalkoxy mit jeweils 1 bis 4 Kohlenstoffatomen steht, und

$R^{11}$ für Alkyl mit 1 bis 4 Kohlenstoffatomen steht,

und

$Z^2$ für die nachstehende Gruppierung steht

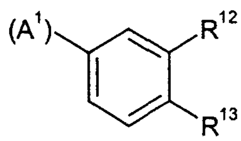

worin

$R^{12}$ für Carboxy oder für jeweils gegebenenfalls durch Cyano, Halogen oder $C_1$-$C_4$-Alkoxy substituiertes Alkoxy oder Alkoxycarbonyl mit jeweils bis zu 4 Kohlenstoffatomen steht und

$R^{13}$ für Cyano, Carbamoyl, Thiocarbamoyl, Halogen, oder für Halogenalkyl oder Halogenalkoxy mit jeweils 1 bis 4 Kohlenstoffatomen steht.

**22.** Substanz gemäß Anspruch 19, **dadurch gekennzeichnet, dass** die Substanz die folgende allgemeine Formel aufweist:

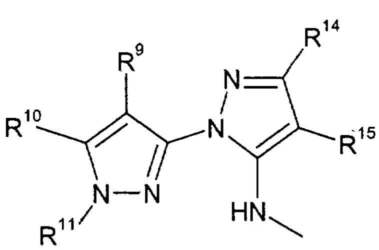

worin

$R^9$, $R^{10}$ und $R^{11}$ die in Anspruch 20 angegebene Bedeutung haben,

$R^{14}$ für Wasserstoff, Halogen oder Alkyl mit 1 bis 4 Kohlenstoffatomen steht, und

$R^{15}$ für Nitro, Cyano, Carbamoyl, Thiocarbamoyl, oder für jeweils gegebenenfalls durch Cyano, Halogen oder $C_1$-$C_4$-Alkoxy substituiertes Alkyl oder Alkoxy mit jeweils 1 bis 4 Kohlenstoffatomen steht.

**23.** Test-System, umfassend

a) Behältnisse mit Mischungen, wie in einem der Ansprüche 1 bis 10 definiert,

b) eine Vorrichtung zum Bestrahlen der Mischungen mit linear polarisiertem Licht einer Wellenlänge, das die fluoreszierende Substanz zur Emission von Licht anregt,

c) eine Vorrichtung zum Messen der Fluoreszenzpolarisationswerte oder der Anisotropiewerte des emittierten Lichts.

**24.** Verwendung einer Substanz gemäß einem der Ansprüche 11 bis 22 für die Fluoreszenz-Korrelations-Spektroskopie.

**25.** Substanzen, die mit dem Enzym Protoporphyrinogen-Oxidase wechselwirken und durch das Verfahren gemäß einem der Ansprüche 1 bis 10 identifiziert wurden.

**Fig. 1**

Struktur von Tracer A

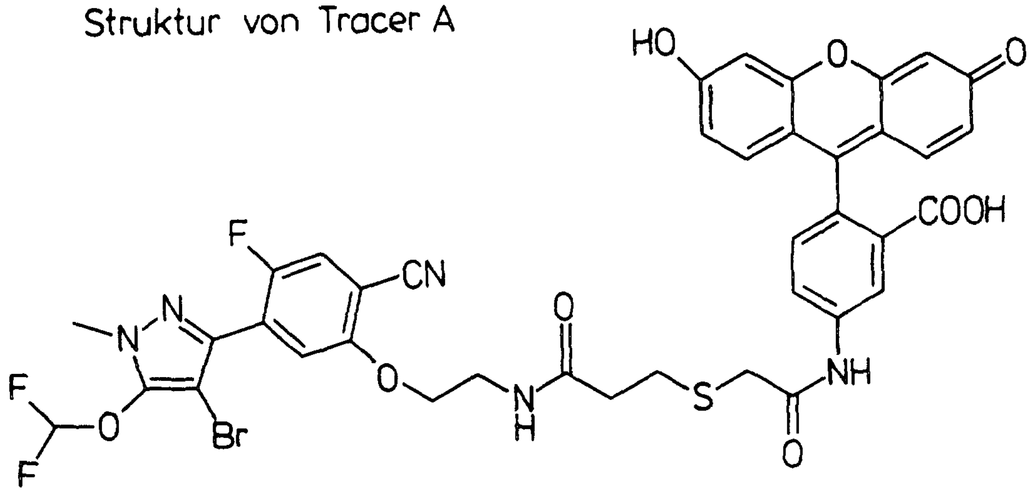

**Fig. 2**

Struktur von Tracer B

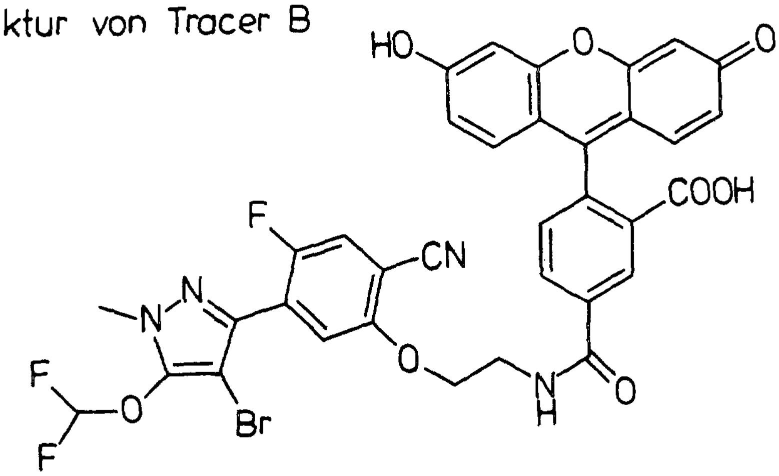

**Fig. 3**

Bindung von Tracer A und B an PPO
Konzentration des Enzymextrakts aus Gerste: 1000mg/ml

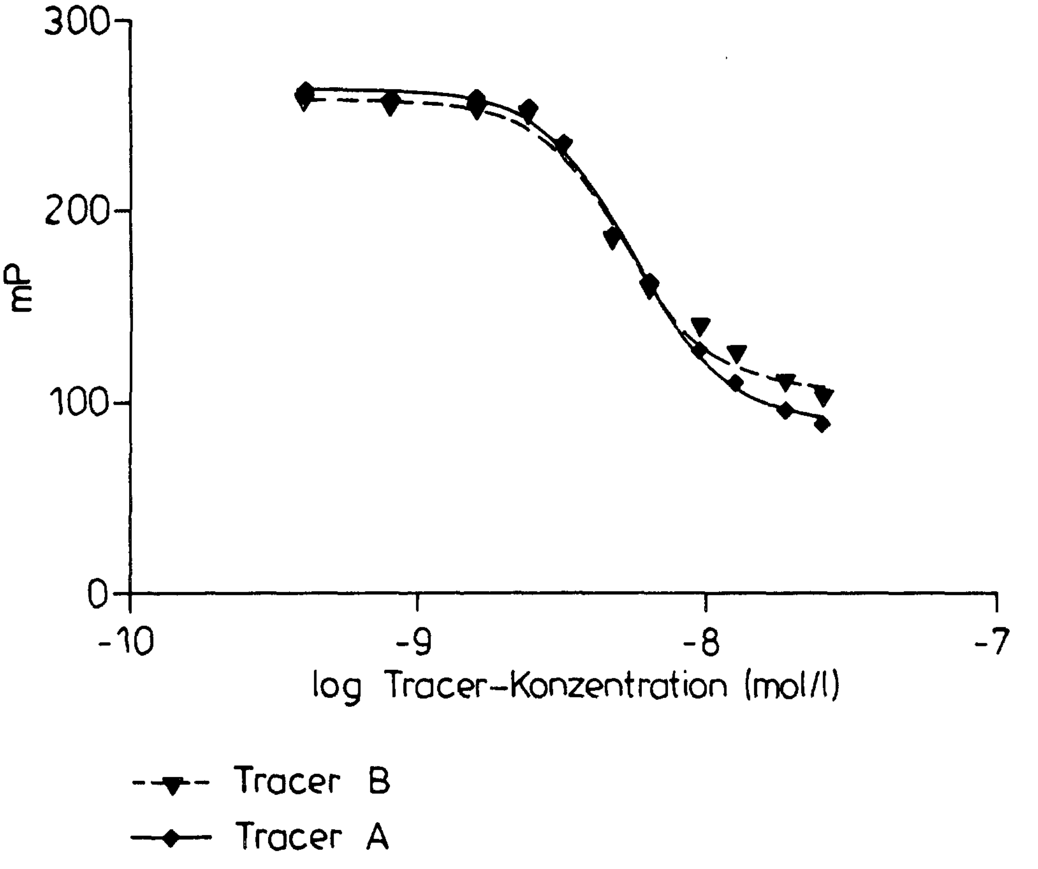

**Fig. 4**

Verdrängung von Tracer A durch eine steigende Konzentration von Wirkstoff. Die eingesetzte Tracer-Konzentration war 1,25nM

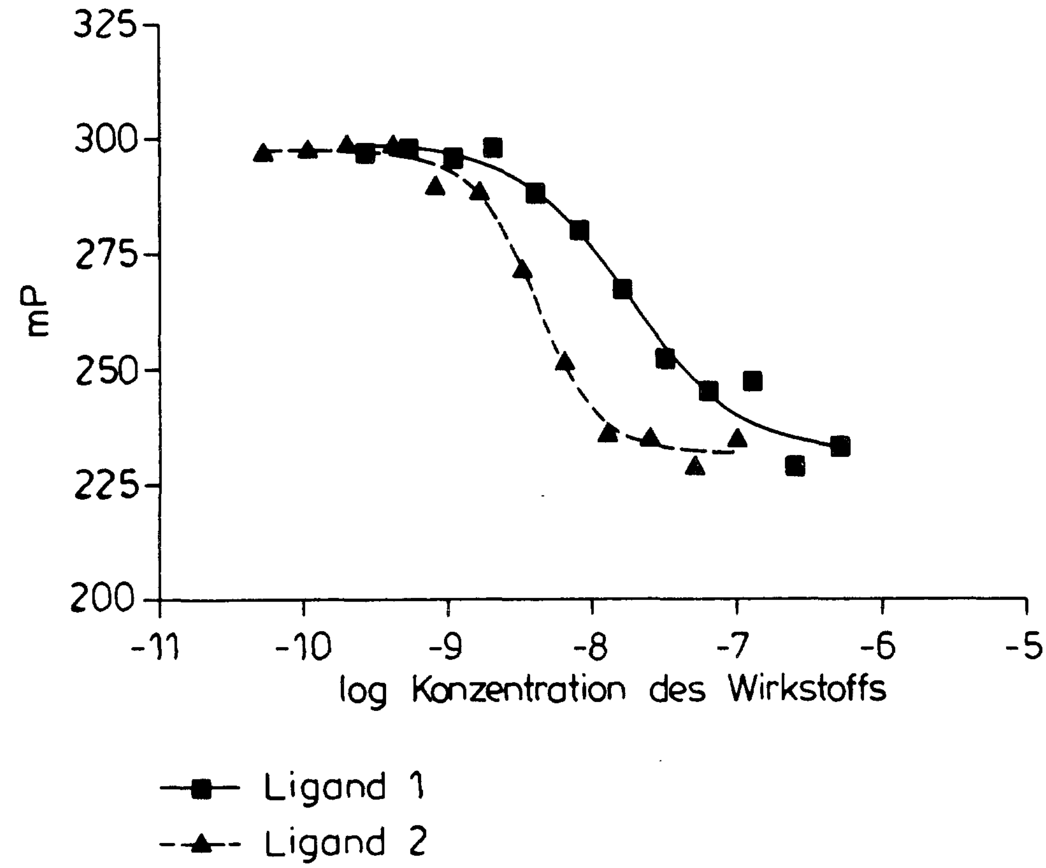


Ligand 1: 5-Amino-1-(2,6-dichlor-4-trifluoromethyl-phenyl)-nitro-1H-pyrazol

Ligand 2: 2-(2-Amino-ethoxy)-4-(4-brom-5-difluormethoxy-1-methyl-1H-pyrazol-3-yl)-5-fluor-benzonitril.